# EUROPÄISCHE PATENTSCHRIFT

(11) **EP 3 070 082 B1**
(45) Veröffentlichungstag und Bekanntmachung des Hinweises auf die Patenterteilung: **09.05.2018**
(21) Anmeldenummer: 16160416.0
(22) Anmeldetag: 15.03.2016
(51) Int. Cl.: C07C 41/09

(54) **VERFAHREN ZUR HERSTELLUNG VON CHLORACETALDEHYDACETALEN**
PROCESS FOR PREPARING CHLOROACETALDEHYDE ACETALS
PROCÉDÉ DE FABRICATION D'ACÉTALES DE CHLORACÉTALDÉHYDE

(30) Priorität: 18.03.2015 DE 102015204901
(43) Veröffentlichungstag der Anmeldung: 21.09.2016
(73) Patentinhaber: Wacker Chemie AG, 81737 München (DE)
(72) Erfinder: Krüger, Benno, 84508 Burgkirchen (DE); Döring, Wolfgang, 84561 Mehring (DE); Fleischmann, Gerald, 84489 Burghausen (DE); Petersen, Hermann, 84489 Burghausen (DE)
(74) Vertreter: Potten, Holger

(56) Entgegenhaltungen:
- DE-B- 1 235 880
- GELAS ET AL: "Cyclic Acetals. XI N[deg.] 544. - Recherches dans la série des acetals cycliques. XI (*). - Hydroxyalkyl-2 et hydroxymethyl-5 dioxannes-1,3:liaison hydrogene intramoléculaire; réactions d'hetérobicyclisation", BULLETIN DE LA SOCIETE CHIMIQUE DE FRANCE. MEMOIRES, MASSON, PARIS, FR, Nr. 9, 1. Januar 1972 (1972-01-01), Seiten 3471-3479, XP008180437, ISSN: 0366-3132
- XUEZHENG LIANG ET AL: "Novel carbon-based strong acid catalyst from starch and its catalytic activities for acetalization", JOURNAL OF MATERIALS SCIENCE, KLUWER ACADEMIC PUBLISHERS, BO, Bd. 46, Nr. 16, 7. April 2011 (2011-04-07) , Seiten 5345-5349, XP019907402, ISSN: 1573-4803, DOI: 10.1007/S10853-011-5472-1
- MESKENS: "Methods for the Preparation of Acetals from Alcohols or Oxiranes and Carbonyl Compounds", SYNTHESIS, 1981, Seiten 501-522, XP002760399,

## Beschreibung

Gegenstand der Erfindung ist ein Verfahren zur Herstellung von acyclischen und cyclischen Chloracetaldehydacetalen aus wässrigen Chloracetaldehyd-Lösungen, durch azeotrope Entfernung von Wasser mit Hilfe eines geeigneten Lösemittels.

Es ist bekannt, dass man Chloracetaldehydacetale durch Chlorieren von Vinylverbindungen (Vinylacetat, Vinylchlorid, Vinylether) in alkoholischen Medien herstellen kann. Eine Diskussion dieses Stands der Technik findet sich in US4440959.

EP0456157B1 beschreibt die Herstellung von Chloracetaldehydacetalen aus dem Trimeren des Chloracetaldehyds in guter Qualität und Ausbeute. Das für die Herstellung eingesetzte Trimere des Chloracetaldehyds ist in EP0368613B1 beschrieben. Dieses Verfahren zur Herstellung des benötigten Trimeren des Chloracetaldehyds ist sehr aufwendig, benötigt große Mengen Lösemittel und konzentrierte Schwefelsäure und hat eine schlechte Ausbeute (ca. 50%), so dass dieses Verfahren technisch nicht relevant ist.

Es ist ferner bekannt, dass Acetale mit Hilfe des Fischer'sehen Acetalisierungsverfahrens, also die direkte Umsetzung des Aldehyds mit Alkoholen in Gegenwart eines sauren Katalysators und der Entfernung des Reaktionswassers mit Hilfe der azeotropen Destillation (Meskens, Synthesis 501-522 (1981) hergestellt werden können, jedoch ist dies Verfahren für niedrigsiedende Aldehyde bedeutungslos, da die Wasserentfernung unvollständig ist oder ausbleibt und deshalb Trockenmittel (z.B. Calciumchlorid usw.) zugesetzt werden müssen. Ähnlich ist das in DE1235880B beschriebene Herstellungsverfahren aus 80%igem Chloracetaldehyd und Alkoholen, bei dem die Wasserentfernung ausschließlich über wasserbindende Mittel erfolgt. Diese verteuern jedoch nicht nur die Herstellung, sondern bilden auch eine erhebliche Menge Abfall, die im Falle der chlorierten Aldehyde aufgrund des Gehalts an chlorierten Bestandteilen, nur schwer zu entsorgen sind. Zudem ist 80%iges Chloracetaldehyd schwer herstellbar und aufgrund des Zersetzungspotentials auch schlecht handhabbar.

GELAS ET AL: "Cyclic Acetals. XI N[deg.] 544. - Recherches dans la Serie des acetals cycliques. XI (*). - Hydroxyalkyl-2 et hydroxymethyl-5 dioxannes-1,3:liaison hydrogene intramoleculaire; reactions d'heterobicyclisation", BULLETIN DE LA SOCIETE CHIMIQUE DE FRANCE. MEMOIRES, MASSON, PARIS, FR, Nr. 9, 1. Januar 1972 (1972-01-01), Seiten 3471-3479, XP008180437 beschreiben die Herstellung cyclischer Chloracetaldehydacetale in Benzol unter Einsatz von wasserfreiem Chloracetaledhyd mit einer Ausbeute von 90% bzw. 70%. Wasserfreies Chloracetaldehyd ist aufgrund seiner thermischen Instabilität in technischem Maßstab nicht bzw. nicht sicher handhabbar.

Xuezheng Liang, Chunqing Li, Chenze Qi, J. Mater Sci (2011) 46:5345-5349 beschreiben u.a. die Herstellung cyclischer Chloracetaldehydacetale durch azeotrope Destillation mit Hilfe von Cyclohexan in einer Dean-Stark Apparatur aus Chloracetaldehyd und den entsprechenden Diolen in Ausbeuten von 99%. Es gibt jedoch keine Angabe, in welcher Form Chloracetaldehyd eingesetzt wird und die maximal beschriebene Ansatzgröße beträgt nur 0,1 mol Chloracetaldehyd und nur 10 ml Cyclohexan als Wasserschlepper. Nach eigener Erkenntnis ist Cyclohexan für azeotrope Acetalisierung von Chloracetaldehyd ungeeignet da es Chloracetaldehyd gut aus dem Reaktionsgemisch entfernt (vgl. Vergleichsbeispiel 3).

Chloracetaldehyd ist in wässriger Lösung (bis 45% Chloracetaldehyd) gut und in hoher Reinheit verfügbar. Es ist jedoch kein technisch relevantes Verfahren bekannt, das geeignet ist, aus wässrigen Chloracetaldehyd-Lösungen Chloracetaldehydacetale herzustellen. Bekannt sind lediglich Verfahren, bei denen man von Chloracetaldehyd-Hemihydrat ausgeht, welches jedoch schwierig herstellbar und aufgrund der Möglichkeit von Zersetzung gefährlich zu handhaben ist.

Aufgabe der Erfindung ist es ein Verfahren zur Herstellung von Chloracetaldehydacetalen einwertiger zweiwertiger oder höherwertiger aliphatischer Alkohole aus Chloracetaldehyd zur Verfügung zu stellen.

Die Aufgabe wird gelöst durch ein Verfahren, bei dem aus einer wässrigen Chloracetaldehyd-Lösung in Gegenwart des zu acetalisierenden Alkohols und eines sauren Katalysators durch azeotrope Entfernung von Wasser mit Hilfe eines Lösemittels das Chloracetaldehydacetal gewonnen wird, dadurch gekennzeichnet, dass das Lösemittel ein einfach oder mehrfach chlorierter oder fluorierter Kohlenwasserstoff mit 1 bis 5 Kohlenstoffatomen, der unter Normaldruck (101,3 kPa) mit Wasser ein Azeotrop mit einem Siedepunkt zwischen 25°C und 80°C bildet, ist.

Bei höherwertigen Alkoholen worunter vorzugsweise 3 bis 5 wertige Alkohole zu verstehen sind, können freie Hydroxylgruppen im Chloracetaldehydacetal verbleiben oder zwei oder mehr Chloracetaldehydacetalgruppen im Molekül gebildet werden.

Unter mehrfach halogenierten Kohlenwasserstoffen sind vorzugsweise 2 bis 4-fach halogenierte Kohlenwasserstoffe zu verstehen.

Besonders bevorzugt handelt es sich bei dem halogenierten Lösemittel um Trichlormethan.

Bei der wässrigen Chloracetaledhydlösung handelt es sich vorzugsweise um eine wässriger Lösung mit 5% bis 70 Gew.%, bevorzugt 30% bis 50 Gew.% Chloracetaldehyd. Derartige Lösungen sind gut und in hoher Reinheit verfügbar.

Bei dem zu acetalisierenden Alkohol handelt es sich vorzugsweise um einen einwertigen zweiwertigen oder höherwertiger aliphatischen Alkohol mit ein bis zwölf C-Atomen, besonders bevorzugt handelt es sich um einen einwertigen oder zweiwertigen aliphatischen Alkohol mit ein bis zwölf C-Atomen.

Die einwertigen aliphatischen Alkohole mit ein bis zwölf C-Atomen können gesättigt oder ungesättigt sein und aromatische Gruppen, oder unter den Reaktionsbedingungen stabile Substituenten (wie z. B. Ether) enthalten.

Die zweiwertigen aliphatischen Alkohole mit ein bis zwölf C-Atome können gesättigt oder ungesättigt sein und aromatische Gruppen oder unter Reaktionsbedingungen stabile Substituenten wie z.B. Ether enthalten. Die Hydroxygruppen können in alpha-, beta- oder gamma-Position zueinander stehen.

Die höherwertigen aliphatischen Alkohole mit ein bis zwölf C-Atomen können gesättigt oder ungesättigt sein und aromatische Gruppen, oder unter den Reaktionsbedingungen stabile Substituenten (wie z.B. Ether) enthalten.

Bevorzugt handelt es sich bei dem einwertigen aliphatischen Alkohol um Methanol, Ethanol, n-Propanol, i-Propanol, 1-Butanol, 2-Butanol, iso-Butanol, tert.-Butanol, 1-Pentanol, Cyclohexanol, Cyclopentanol.

Bevorzugt handelt es sich bei dem zweiwertigen aliphatischen Alkohol um 1,2-Ethandiol, 1,2-Propandiol, 1,3-Propandiol, 1,4-Butandiol, 2,3-Butandiol, 1,3-Hexandiol, cis-1,3-Cyclohexandiol, cis-1,2-Cyclohexandiol.

Bevorzugt handelt es sich bei dem höherwertzigen aliphatischen Alkohol um Glycerin, Pentaerythrit, Xylitol.

Der saure Katalysator ist vorzugsweise eine anorganische oder organische Säure, wie z.B. Salzsäure, Schwefelsäure, Phosphorsäure, Methansulfonsäure, Toluolsulfonsäure, Trifluormethansulfonsäure, Trifluoressigsäure, Benzolphosphonsäure, oder ein stark saueres Ionenaustauscherharz. Unter einem stark sauren Ionenaustauscherharz ist vorzugsweise ein Ionenaustauscherharz mit Sulfonsäuregruppen zu verstehen. Derartige stark saure Ionenaustauscherharze sind kommerziell erhältlich unter der Bezeichnung Dowex® oder Amberlite®.

Besonders bevorzugt handelt es sich um Phosphorsäure, Toluolsulfonsäure, Schwefelsäure oder Dowex®.

Bei dem Chloracetaldehydacetal handelt es sich vorzugsweise um acyclischen oder cyclische Chloracetaldehydacetale mit ein bis zwölf C-Atomen.

Vorzugweise liegt das Lösemittel im Reaktionsansatz in einem Volumenverhältnis von 1:10 bis 5:1 Lösemittel bezogen auf Chloracetaldehydlösung vor.

Der saure Katalysator wird vorzugsweise in einer Menge von 0,01 Mol % bis 2 Mol % bezogen auf das eingesetzte Chloracetaldehyd eingesetzt. Aufgrund des sauren pH-Wertes der Chloracetaldehydlösung ist auch eine Reaktion ohne Katalysator möglich, jedoch ist dann die Reaktionsgeschwindigkeit für einen technischen Prozess zu langsam.

Überraschenderweise können Chloracetaldehydacetale in hoher Ausbeute und hoher Reinheit auf einfache Weise durch Entwässerung wässriger Chloracetaldehyd-Lösungen mit einem halogenierten Lösemittel in Gegenwart des zu acetalisierenden Alkohols und eines sauren Katalysators gewonnen werden. Dies ist selbst bei niedrigsiedenden Alkoholen möglich. Bei säureempfindlichen Chloracetaldehydacetalen muss der saure Katalysator vor der Destillation neutralisiert werden. Dies geschieht vorzugsweise mittels Aminen deren Siedepunkt deutlich höher liegt als der Siedepunkt des Produkts. Es können auch Alkali oder Erdalkalicarbonate, -hydrogencarbonate oder -oxide verwendet werden.

Die Verwendung aliphatischer Kohlenwasserstoffe wie Hexan, Pentan, Cyclohexan als Lösemittel erwies sich als ungeeignet, da hier große Mengen des Chloracetaldehyds in den Wasserabscheider ausgetragen werden und somit die Ausbeute an Chloracetaldehydacetal drastisch vermindert wird. Insbesondere bei niedrig siedenden Alkoholen, worunter im Rahmen dieser Erfindung vorzugsweise Alkohole mit einem Siedepunkt von 65°C bis 80°C unter Normalbedingungen zu verstehen sind, erwies es sich als vorteilhaft, wenn das als Schlepper verwendete Lösemittel nur geringe Mengen des Alkohols, worunter vorzugsweise Mengen von weniger als 20 Gew.% zu verstehen sind, aus dem Reaktionsgemisch entfernt. Versuche im Rahmen der vorliegenden Erfindung haben überraschenderweise ergeben, dass hinsichtlich der Verwendung als Lösemittel einfach oder mehrfach halogenierte Kohlenwasserstoffe mit ein bis fünf C-Atomen, die unter Normaldruck mit Wasser ein Azeotrop mit einem Siedepunkt zwischen 25°C und 80°C bilden bevorzugt sind. Als besonders günstig erwies sich Trichlormethan. Dieses entfernt aufgrund von Wechselwirkungen zwischen chlorierten Molekülen in erheblich geringerem Ausmaß Chloracetaldehyd aus dem Reaktionsgemisch.

Ein Vorteil des erfindungsgemäßen Verfahrens besteht darin, dass das zum Auskreisen von Wasser eingesetzte Lösemittel bzw., bei niederen Alkoholen, wie Methanol, Ethanol oder isoPropanol, das Gemisch aus Lösemittel und dem entsprechenden Alkohol, ohne weitere Reinigung wieder im erfindungsgemäßen Verfahren eingesetzt werden kann. Ein weiterer Vorteil ist, dass außer geringen Mengen Destillationsrückstand kaum Abfälle, anfallen (z.B. in Beispiel 1 und 6 <2% der eingesetzten Menge).

Die folgenden Beispiele dienen der weiteren Erläuterung der Erfindung.

### Beispiel 1: Herstellung 2-Chlormethyl-1,3-dioxepan

In einen 4 1 Vierhalskolben mit Wasserabscheider, Rückflusskühler, KPG-Rührer und Innenthermometer wurden 1744,4 g 45%ige wässrige Chloracetaldehyd-Lösung (10 mol Chloracetaldehyd), 919,0 g (10,2 mol) 1,4-Butandiol, 11 Trichlormethan und 2 g p-Toluolsulfonsäure gegeben. Die zweiphasige Mischung wurde unter Rühren am Wasserabscheider zum Rückfluss erhitzt, bis sich kein Wasser mehr abscheidet (ca. 10 h). Das Reaktionsgemisch wurde dabei einphasig. Danach wurde das Reaktionsgemisch im Vakuum (15 mbar, 80°C) destilliert.
Ausbeute: 1459,3 g (96,9%), Reinheit >99% (GC)

### Vergleichsbeispiel 1

Der Versuch wurde wie in Beispiel 1 beschrieben durchgeführt mit dem Unterschied, dass anstatt Trichlormethan n-Hexan verwendet wurde.
Ausbeute 873,5 g (58%), Reinheit >99% (GC)

### Beispiel 2: Herstellung von Chloracetaldehyddiethylacetal

In einem 6 1 Vierhalskolben mit Wasserabscheider, Rückflusskühler, Füllkörperkolonne, KPG-Rührer und Innenthermometer und Dosiereinheit wurden 2442 g 45%ige wässrige Chloracetaldehyd-Lösung (14 mol Chloracetaldehyd), 1612 g (35 mol) Ethanol, 1500 ml Trichlormethan und 2,8 g p-Toluolsulfonsäure gegeben. Die zweiphasige Mischung wurde unter Rühren am Wasserabscheider zum Rückfluss erhitzt bis sich kein Wasser mehr abscheidet (7 h). Das Reaktionsgemisch wird dabei einphasig. Danach wurden weitere 322,5 g (7 mol) Ethanol unter Rückfluss und Wasserabscheiden zudosiert. Anschließend wurden nochmals 253,7 g (4,35 mol) Ethanol sowie 500 g Trichlormethan zugegeben und am Wasserabscheider zum Rückfluss erhitzt bis sich kein Wasser mehr abschied. Anschließend werden 31,2 g Tributylamin zur Neutralisation zugesetzt und das Reaktionsgemisch im Vakuum (100 mbar, 90°C) fraktioniert destilliert.
Ausbeute 1987 g (93,0% der Theorie) Reinheit > 99% (GC)

### Beispiel 3: Herstellung von 2-Chlormethyl-1,3-dioxolane

In einem 11 Vierhalskolben mit Wasserabscheider, Rückflusskühler, KPG-Rührer und Innenthermometer und Dosiereinheit wurden 174,44 g 45%ige wässrige Chloracetaldehyd-Lösung (1 mol Chloracetaldehyd), 68,28 g (1,1 mol) Ethylenglycol, 200 ml Trichlormethan und 0,2 g p-Toluolsulfonsäure gegeben. Die zweiphasige Mischung wurde unter Rühren am Wasserabscheider zum Rückfluss erhitzt bis sich kein Wasser mehr abscheidet (insgesamt ca. 7 h). Das Reaktionsgemisch wird dabei einphasig. Danach werden 2,2 g Tributylamin zur Neutralisation der p-Toluolsulfonsäure zugesetzt und das Reaktionsgemisch im Vakuum (75 mbar, 85°C) destilliert.
Ausbeute 111,6 g (91%), Reinheit > 99% (GC)

### Beispiel 4: Herstellung von Chloracetaldehyddimethylacetal

In einem 1 1 Vierhalskolben mit Wasserabscheider, Rückflusskühler, KPG-Rührer und Innenthermometer und Dosiereinheit wurden 174,44 g 45%ige wässrige Chloracetaldehyd-Lösung (1 mol Chloracetaldehyd), 16,02 g (0,5 mol) Methanol, 200ml Trichlormethan und 0,2 g p-Toluolsulfonsäure gegeben. Die zweiphasige Mischung wurde unter Rühren am Wasserabscheider zum Rückfluss erhitzt, bis sich kein Wasser mehr abscheidet. Danach wurden weitere 48 g (1,5 mol) Methanol unter Rückfluss und Wasserabscheiden zudosiert (insgesamt ca. 7 h). Das Reaktionsgemisch wird dabei einphasig. Danach werden 2,2 g Tributylamin zur Neutralisation der p-Toluolsulfonsäure zugesetzt und das Reaktionsgemisch im Vakuum (100 mbar, 90°C) destilliert.
Ausbeute 99,7 g (80%), Reinheit > 99% (GC)

### Vergleichsbeispiel 2

Der Versuch wurde wie in Beispiel 4 beschrieben durchgeführt mit dem Unterschied, dass anstatt Trichlormethan n-Hexan verwendet wurde. Der Versuch wurde nach 4 h abgebrochen, da Methanol und Chloracetaldehyd zum großen Teil aus dem Reaktionsgemisch in die wässrige Phase des Wasserabscheiders ausgetragen waren.

### Vergleichsbeispiel 3

Der Versuch wurde wie in Beispiel 4 beschrieben durchgeführt mit dem Unterschied, dass anstatt Trichlormethan Cyclohexan verwendet wurde. Der Versuch wurde nach 4 h abgebrochen, da Methanol und Chloracetaldehyd zum großen Teil aus dem Reaktionsgemisch in die wässrige Phase des Wasserabscheiders ausgetragen waren.

### Beispiel 5: Herstellung von Chloracetaldehyddimethylacetal

Der Versuch wurde wie in Beispiel 4 beschrieben durchgeführt mit dem Unterschied, dass anstatt 2 mol Methanol 4 mol Methanol eingesetzt wurden (1,5 mol vorgelegt und 2,5 mol dosiert).
Ausbeute: 109,1 g (87.6%), Reinheit > 99% (GC)

### Beispiel 6: Herstellung von Chloracetaldehyddimethylacetal

In einem 1 l Vierhalskolben mit Wasserabscheider, Rückflusskühler, KPG-Rührer und Innenthermometer und Dosiereinheit wurden 200ml Trichlormethan und 0,2 g Toluolsulfonsäure gegeben. Die Mischung wurde unter Rühren am Wasserabscheider zum Rückfluss erhitzt und bei Eintreten des Rückflusses eine Mischung aus 348,9 g 45%ige wässrige Chloracetaldehyd-Lösung (2 mol Chloracetaldehyd), 96,12 g (3 mol) Methanol über einen Zeitraum von 6h zudosiert. Danach wurden weitere 96,12g (3 mol) Methanol über einen Zeitraum von 2 Stunden zudosiert.
Ausbeute: 209,6 g (84,1%), Reinheit > 99% (GC)

### Beispiel 7: Herstellung von Chloracetaldehyddimethylacetal

In einem 6 1 Vierhalskolben mit Wasserabscheider, Rückflusskühler, Füllkörperkolonne, KPG-Rührer und Innenthermometer und Dosiereinheit wurden 3140 g 45%ige wässrige Chloracetaldehyd-Lösung (18 mol Chloracetaldehyd), 865 g (27 mol) Methanol, 1500ml Trichlormethan und 3,6 g p-Toluolsulfonsäure gegeben. Die zweiphasige Mischung wurde unter Rühren am Wasserabscheider zum Rückfluss erhitzt. Während des Wasserabscheidens wurden weitere 1153g (36 mol) Methanol sowie 500 ml Trichlormethan zudosiert wobei die Reaktionsmischung einphasig wurde. Sobald sich kein Wasser mehr Abschied, wurden 40,14g Tributylamin zur Neutralisation zugegeben und das Reaktionsgemisch im Vakuum (80mbar, 63°C) fraktioniert destilliert.
Ausbeute 1900g (88,8% der Theorie) Reinheit > 99% (GC)

## Patentansprüche

1. Verfahren zur Herstellung von Chloracetaldehydacetalen einwertig, zweiwertig oder höherwertiger aliphatischer Alkohole bei dem aus einer wässrigen Chloracetaldehyd-Lösung in Gegenwart des zu acetalisierenden Alkohols und eines sauren Katalysators durch azeotrope Entfernung von Wasser mit Hilfe eines Lösemittels das Chloracetaldehydacetal gewonnen wird, **dadurch gekennzeichnet, dass** das Lösemittel ein einfach oder mehrfach chlorierter oder fluorierter Kohlenwasserstoff mit ein bis fünf C-Atomen ist, der unter Normaldruck mit Wasser ein Azeotrop mit einem Siedepunkt zwischen 25°C und 80°C bildet.

2. Verfahren nach Anspruch 1 **dadurch gekennzeichnet, dass** das halogenierte Lösemittel Trichlormethan ist.

3. Verfahren nach einem der Ansprüche 1 oder 2, **dadurch gekennzeichnet, dass** der zu acetalisierenden Alkohol ein einwertiger, zweiwertiger oder höherwertiger aliphatischer Alkohol mit ein bis zwölf C-Atomen ist.

4. Verfahren nach einem der Ansprüche 1 bis 3, **dadurch gekennzeichnet, dass** der saure Katalysator eine anorganische oder organische Säure, oder ein stark saures Sulfonsäuregruppen haltiges Ionenaustauscherharz ist.

5. Verfahren nach einem der Ansprüche 1 bis 4, **dadurch gekennzeichnet, dass** der saure Katalysator in einer Menge von 0,01 Mol% bis 2 Mol% bezogen auf das eingesetzte Chloracetaldehyd vorliegt.

## Claims

1. Process for preparing chloroacetaldehyde acetals of monohydric, dihydric or higher-functionality aliphatic alcohols, in which the chloroacetaldehyde acetal is obtained from an aqueous chloroacetaldehyde solution in the presence of the alcohol to be acetalized and an acid catalyst by azeotropic removal of water with the aid of a solvent, **characterized in that** the solvent is a singly or multiply chlorinated or fluorinated hydrocarbon which has from one to five carbon atoms and forms an azeotrope having a boiling point in the range from 25°C to 80°C with water under atmospheric pressure.

2. Process according to Claim 1, **characterized in that** the halogenated solvent is trichloromethane.

3. Process according to either of Claims 1 and 2, **characterized in that** the alcohol to be acetalized is a monohydric, dihydric or higher-functionality aliphatic alcohol having from one to twelve carbon atoms.

4. Process according to any of Claims 1 to 3, **characterized in that** the acid catalyst is an inorganic or organic acid or a strong acid ion exchange resin containing sulfonic acid groups.

5. Process according to any of Claims 1 to 4, **characterized in that** the acid catalyst is present in an amount of from 0.01 mol% to 2 mol% based on the chloroacetaldehyde used.

## Revendications

1. Procédé de fabrication de chloroacétaldéhyde-acétals d'alcools aliphatiques monovalents, bivalents ou d'une valence supérieure, selon lequel, le chloroacétaldéhyde-acétal est obtenu à partir d'une solution aqueuse de chloroacétaldéhyde en présence de l'alcool à acétaliser et d'un catalyseur acide, par élimination azéotropique d'eau à l'aide d'un solvant, **caractérisé en ce que** le solvant est un hydrocarbure chloré ou fluoré une fois ou plusieurs fois contenant un à cinq atomes C, qui forme à pression normale avec l'eau un azéotrope ayant un point d'ébullition compris entre 25 °C et 80 °C.

2. Procédé selon la revendication 1, **caractérisé en ce que** le solvant halogéné est le trichlorométhane.

3. Procédé selon l'une quelconque des revendications 1 ou 2, **caractérisé en ce que** l'alcool à acétaliser est un alcool aliphatique monovalent, bivalent ou d'une valence supérieure, contenant un à douze atomes C.

4. Procédé selon l'une quelconque des revendications 1 à 3, **caractérisé en ce que** le catalyseur acide est un acide inorganique ou organique, ou une résine échangeuse d'ions fortement acide contenant des groupes acide sulfonique.

5. Procédé selon l'une quelconque des revendications 1 à 4, **caractérisé en ce que** le catalyseur acide est présent en une quantité de 0,01 % en moles à 2 % en moles, par rapport au chloroacétaldéhyde utilisé.
